# EUROPEAN PATENT APPLICATION

(11) **EP 2 000 119 A1**
(43) Date of publication of application: **10.12.2008**
(21) Application number: 07394016.5
(22) Date of filing: 08.06.2007
(51) Int. Cl.: A61F 13/02, A61K 9/70, A61F 17/00

(54) **Wound dressings**

(71) Applicant: Royal College of Surgeons in Ireland, Dublin 2 (GB)
(72) Inventor: The designation of the inventor has not yet been filed
(74) Representative: Purdy, Hugh Barry

(57) **Abstract**

A wound dressing for a wound of the type having a wound bed and wound exudate comprises an absorbent core disposed on the dressing such that upon application to a wound the absorbent core will absorb wound exudate from the wound bed, and a wound healing substance around the absorbent core which is disposed on the dressing such that upon application to a wound, the wound healing substance will be brought into contact with a periphery of the wound bed. Also described is a kit, and a method, for treating a wound which kit and method employ a wound dressing according to the invention.

## Description

### Technical Field

The invention relates to a wound dressing for a wound of the type having a wound bed and wound exudate. In particular, the invention relates to wound dressings for chromic wounds such as those associated with vascular disease, diabetes and prolonged bed-rest.

### Background Art

Chronic wounds are a serious debilitating complication of vascular disease, diabetes and prolonged bed-rest. In diabetes chronic wounds occur as ulcers on the lower extremities. In the United States, the annual cost of treatment of diabetes-related chronic wounds is estimated to be $16-21 billion. The global market for wound care products alone is in the region of $4.2 - $6.9 billion per annum. This market accounts for products which can be broadly divided into three different categories. The simplest form being standard absorbent dry bandages which cover and protect the wound while absorbing wound exudates. The second category is moist dressings, which promote a moist environment for the wound, and which have been proven to accelerate healing in certain hard-to-heal or chronic wounds.

More advanced technologies provide the ideal healing properties for different types of wounds and the substances that have been developed for this purpose include hydrocolloids, foams, hydrogels and alginates. Bandages and wound dressings alone to not directly enhance wound healing. Generally, their role can be seen as passive by providing an environment conducive to the healing process. Attempts to stimulate wound healing have employed the application of cytokines and growth factors, although results have been mixed. Regranex (Johnson & Johnson) is the only marketed product from such research.

### Statements of Invention

According to one aspect of the invention, there is provided a wound dressing for a wound of the type having a wound bed and wound exudate, the dressing comprising an absorbent core disposed on the dressing such that upon application to a wound the absorbent core will absorb wound exudate from the wound bed, the dressing incorporating a wound healing substance around the absorbent core which is disposed on the dressing such that upon application to a wound, the wound healing substance will be brought into contact with a periphery of the wound bed. Use of the wound dressing of the invention has a two-fold effect; removal of the exudate hinders the development of bacterial infections in the wound bed; and the provision of a wound healing composition around the absorbent core delivers the wound promoting component to where it is needed, the advancing wound edge.

In this specification, the term "periphery of the wound bed" should be understood as meaning the border of the wound bed, or that part of the wound bed adjacent the periphery. Thus, the dressing of the invention is adapted to apply wound healing substance to the border of the wound bed, i.e. to the edge of the wound bed itself, or on the wound bed adjacent to the wound edge.

In this specification, the term "absorbent core" should be understood as meaning a formation which is specifically adapted for absorbing wound exudate from a wound bed. Examples of materials that are suitable for forming absorbent cores will be known to those skilled in the art. Specific examples are provided below.

In this specification, the term "wound healing substance" should be understood as meaning any substance or formulation which has the function of actively promoting the wound healing process. Typically, the substance is selected from the group comprising: a gel; a cream; an ointment; a liquid; and a powder. Suitably, the wound healing substance is a phenytoin-containing composition, such as a gel, a cream, a paste or an emulsion. The phenytoin may be, for example, a phenytoin acid, phenytoin free base, a phenytoin salt, a derivative of phenytoin, or a mixture thereof. In an alternative embodiment, the wound healing substance is REGRANEX. Other examples of wound healing substances will be known to those skilled in the art of wound healing. Generally, the term "wound healing substance" would not encompass passive wound healing agents such as those that provide an environment conducive to wound healing. Ideally, the term excludes rubefacient or hyperaemic substances.

Typcially, the wound healing substance encircles the absorbent core. However, the wound healing substance may be intermittently incorporated around the absorbent core and follow a circular, oval, square, rectangular, or any other shaped path.

Suitably, the wound healing substance is separated from the absorbent core by a liquid impermeable barrier layer.

In one embodiment, the absorbent core has a wound abutting face, wherein a liquid impermeable barrier layer covers the surface of the absorbent core except for the wound abutting face. Ideally, the liquid impermeable barrier layer is gas permeable.

In one embodiment, the wound dressing further includes an odour control layer. Suitable embodiment of wound healing layers will be known to those skilled in the field. For example, the wound control layer may comprise a material that has odour absorbing properties, i.e. activated charcoal, zeolites, bentonite, etc. Typically, the odour control layer is disposed on an opposite side of the absorbent core to the wound bed. Suitably, the wound healing substance is separated from the odour control layer by means of a liquid impermeable barrier, suitably a gas permeable barrier.

In a preferred embodiment, the absorbent core has a central wicking portion which in use abuts (i.e. is in contact with, or lies adjacent to, such that it is in liquid communication therewith) the wound bed, and a main body portion located above, and in liquid communication with, the central wicking portion. Suitably, the absorbent core has a generally T-shaped profile. This has the function of providing a lower wicking portion that "pulls" exudate away from the wound bed, and transports the exudate up the wicking portion to the main body portion where the exudate is distributed laterally. In this regard, the term "T-shaped profile" should be understood as meaning that the absorbent core has a wicking stem, and an upper exudate storage part having a body that is wider than the wicking stem. Typically, the main body portion of the absorbent core has a surface area greater then a surface area of the central wicking portion.

Suitably, the wound dressing includes a liquid and gas permeable topsheet that in use abuts the wound. Typically, the topsheet comprises a non-woven airlaid material. In one embodiment, the top sheet comprises a central portion formed of a unidirectional material that allows passage of fluid only in the direction of the absorbent core. Such unidirectional materials will be well known to those skilled in the art, and they generally comprise a multiplicity of cone-shaped apertures that allow passage of liquid in one direction.

Suitably, the absorbent core is fixed to the topsheet. When the topsheet comprises a unidirectional material, the absorbent core is suitably fixed to the part of the topsheet that comprises the unidirectional material.

In one embodiment, the wound dressing further comprises a backsheet. Typically, the backsheet is semi-occlusive. This helps to maintain a moist environment in the vicinity of the wound while also allowing access of air to the wound.

In a preferred embodiment, the wound dressing further includes a layer of padding disposed adjacent the backsheet.

The invention also relates to a wound dressing according to the invention and comprising:
- an absorbent core;
- a wound healing substance disposed along a periphery of the absorbent core;
- an odour absorbing layer; and
- a liquid impermeable barrier disposed between the odour absorbing layer and the absorbent core.

The invention also relates to a wound dressing according to the invention and comprising:
- an absorbent core;
- a wound healing substance disposed along a periphery of the absorbent core;
- an odour absorbing layer; and
- a liquid impermeable barrier disposed between the odour absorbing layer and the absorbent core,
wherein the absorbent core has a generally T-shaped profile and comprises a central wicking portion and a main body portion located above, and in liquid communication with, the central wicking portion.

In an alternative embodiment, the invention relates to a wound dressing for a wound of the type having a wound bed and wound exudate, the dressing comprising an absorbent core disposed on the dressing such that upon application to a wound the absorbent core will absorb wound exudate from the wound bed, wherein the absorbent core has a generally T-shaped profile and comprises a central wicking portion which in use abuts the wound bed, and a main body portion located above, and in liquid communication with, the central wicking portion.

The invention also relates to a kit comprising a plurality of wound dressings, at least some of the wound dressings in the kit differing from the others by virtue of the location of the wound healing substance. In this way, a user can apply a wound dressing to a wound when the wound has a given size, and when the wound has partially healed and the wound size has decreased, the use can apply a further wound dressing in which the location of the wound healing substance on the dressing matches the periphery/border of the wound. In one embodiment, an outer surface of the dressing, or a wrapping for an individual dressing, will have a visual indication of the location on the dressing of the wound healing substance. Thus will enable a user to choose a dressing which is suitable for the shape and size of the wound to be dressed.

The invention also provides a method of treating a wound of the type having a wound bed and wound exudate, the method comprising applying a wound dressing according to the invention to the wound such that the wound healing substance in the dressing is brought into contact with the periphery of the wound bed.

The invention also relates to a kit for treating a wound of the type having a wound bed and wound exudate, the kit comprising:
- a wound dressing comprising an absorbent core disposed on the dressing such that upon application to a wound the absorbent core will absorb wound exudate from the wound bed;
- a wound healing substance contained within a suitable dispenser; and
- instructions for the user to apply the wound healing substance onto the wound dressing around the absorbent core such that, upon application to the wound, the wound healing substance will be brought into contact with a periphery of the wound bed.

Suitably, the wound healing substance of the kit is selected from the group comprising: a gel; a cream; an ointment; a liquid; and a powder.

In one embodiment of the invention, the wound dressing is in two parts, a first part comprising a top sheet which is adapted to be applied to the skin on its own, and a second part which comprises the remaining parts of the wound dressing of the invention. Typically, the top sheet has visual indicators which indicate there the absorbent core, especially the wicking stem of the absorbent core, would be positioned when the second part is overlaid on top of the topsheet. In use, the top sheet is applied to the wound. Then the wound healing substance is applied to the topsheet around that part of the topsheet where the absorbent core will be placed (this may be marked on the topsheet) and around a periphery, or adjacent to a periphery, of the wound. Then, the second part of the wound dressing is placed on top of the topsheet. Suitably, that part of the topsheet which abuts the absorbent core will include an adhesive to enable the absorbent core adhere to the topsheet.

The invention also relates to a method of treating a wound which employs a kit according to the invention, which method comprises the steps of:
- applying a wound healing substance to a wound dressing around the absorbent core in such a disposition such that upon application of the wound dressing to the wound, the wound healing substance will be brought into contact with a periphery of the wound bed; and
- applying the wound dressing with the wound healing substance incorporated thereon on the wound.

The invention also relates to a method of treating a wound of the type having a wound bed and wound exudate, the method employing a wound dressing of the type having an absorbent core disposed on the dressing such that upon application to a wound the absorbent core will absorb wound exudate from the wound bed, the method comprising the steps of applying a wound healing substance to the wound dressing around the absorbent core in such a disposition such that upon application of the wound dressing to the wound, the wound healing substance will be brought into contact with a periphery of the wound bed, and applying the wound dressing with the wound healing substance incorporated thereon on the wound.

The packaging for the wound dressing of the invention, or for the wound dressing kits of the invention, may be designed, or formed of a material, that is resistant to compression. This is especially relevant for wound dressings that include a wound healing gel, as compression of such dressings may result in the gel being squeezed out of the dressing. Further, the packaging should have an overall moisture vapour transmission rate that is suitable for preventing the gel drying out during the time that the dressing would normally be stored and transported.

The invention is primarily directed to wounds having a wound bed and wound exudate. An example of such wounds would be chronic wounds, such as those that are associated with vascular disease, diabetes, and prolonged bed-rest.

### Brief Description of the Figures

The invention will be more clearly understood from the following description of some embodiments thereof, given by way of examples only, in which:
Fig. 1 is a an illustration of a wound dressing according to the invention;
Fig. 2 is an illustration of a wound dressing according to an alternative embodiment of the invention; and
Fig. 3 is an illustration of a wound dressing according to a further embodiment of the invention.

### Detailed Description of the Invention

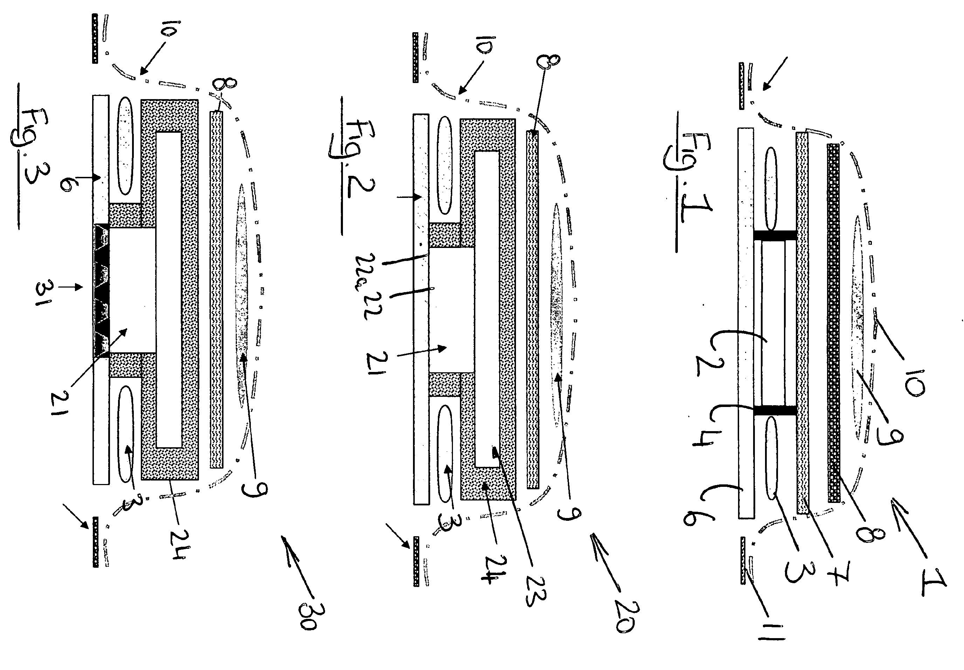

Referring to the figures, an initially to Fig. 1 thereof, there is illustrated a wound dressing according to the invention, and indicated generally by the reference numeral 1. The wound dressing 1 comprises an absorbent core 2 located in the centre of the dressing, and a peripheral layer of wound healing gel 3 incorporated into the dressing around the absorbent core 2. The wound healing gel is formed according to the formulation provided in Example 1 or 2. Between the gel 3 and absorbent core 2 is a barrier 4 which prevent movement of the gel 3 into the absorbent core 2. The gel 3 and absorbent core 2 are sandwiched between an airlaid non-woven topsheet 6 that is permeable to fluids and in use abuts the wound, and a further barrier layer 7 that is liquid impermeable and gas permeable. An odour control layer 8 comprising an activated charcoal impregnated material is located above the barrier layer 7, and a layer of padding 9 is located between the odour control layer 8 and the backsheet 10. Finally, a strip of adhesive 11 is incorporated onto a periphery of the backsheet 10.

In use, the dressing 1 is applied to a wound whereby the layer of wound healing gel 3 is brought into contact with a periphery of the wound, whereby the gel will actively promote wound healing through the action of the phenytoin in the gel. The wound exudate in a centre of the wound bed will pass through the permeable topsheet 6 into the absorbent core 2 by virtue of the wicking action of the absorbent core 2. Movement of the exudate sideways into the gel 3 is prevented by virtue of the liquid impermeable barrier 4, and movement longitudinally into the odour control layer 8 is prevented by the liquid impermeable barrier layer 7. Odours emanating from the wound bed, and from the exudate in the wound bed and in absorbent core, will be able to diffuse through the gas permeable barrier 7 and into the odour control layer 8 where they will be absorbed by the activated charcoal. Typically, the dressing will be worn for a few days until healing has progressed to the extent that the size of the wound has decreased. At this stage, the dressing may be changed, and a new dressing, in which the positioning of the wound healing gel on the dressing substantially matches the periphery of the wound bed, is applied to the wound.

Referring to Fig. 2, there is illustrated an alternative embodiment of the wound dressing, indicated generally by the reference numeral 20, and in which parts similar to those described with reference to the previous embodiment are assigned the same reference numerals. In this embodiment, the absorbent core 21 has a generally T-shaped profile with a wicking stalk 22 having a wound-facing end 22a, and a main body portion 23. The surface of the core 21, with the exception of the wound-facing end 22a, is covered with a liquid impermeable, gas permeable, barrier layer 24, which allows odours, but prevents wound exudate, passing out of the core. The use of this embodiment is substantially the same as that described with reference to Fig. 1 with the exception that the shape of the absorbent core allows for a more efficient removal of exudate from the wound bed. This is partly due to the ability of the wicking stalk to "pull" exudate from the wound bed, and transport it longitudinally to the main body portion 23, where the exudate can be moved laterally throughout the main body portion 23. The main body portion is also located quite close to the odour control layer, thereby facilitating movement of odours from the exudate in the core. Further, the wider, thinner, nature of the core in this embodiment provides for a greater surface area for movement of odours out of the core.

Referring to Fig. 3, there is illustrated a further embodiment of a wound dressing according to the invention, indicated generally by the reference numeral 30, and in which parts similar to those described with reference to the previous embodiments are assigned the same reference numerals. The dressing of this embodiment is substantially identical to the dressing of Fig. 2, with the exception that the topsheet 6 comprises a central portion 31 composed of a unidirectional film. The use of the wound dressing of this embodiment is identical to that of the previous embodiment with the exception that the unidirectional film prevents movement of exudate from the core into the wound bed.

The materials used to make a wound dressing according to the invention will be well known to those skilled in the art. However, examples of the individual components are provided below:

### Wound Healing Composition

### EXAMPLE 1 Extended-release phenytoin gel.

Carbomer 974 PNF 1g is added to approximately 80g deionised water. Following complete addition of Carbomer, the preparation is mixed for 30 minutes. Phenytoin sodium 5g is gradually added while mixing. The gel thickens. The gel is made up to 100g with deionised water, with mixing. The pH is adjusted to 7.4 with sodium hydroxide.

### EXAMPLE 2. Extended-release phenytoin gel.

Hydroxypropyl-β-Cyclodextrin 30g is dissolved in approximately 60g deionised water with stirring. Phenytoin sodium 1g is added slowly to form a suspension. Carbomer 974 PNF 1g is gradually added with mixing at 1500rpm. The gel is made up to 100g with deionised water, with mixing. The pH is adjusted to 7.4 with sodium hydroxide.

### Absorbent Core

The absorbent core is positioned between the backing sheet and the topsheet. Such an absorbent core may essentially comprises a web or batt of hydrophilic fiber material. Examples of hydrophilic fiber material include, cellulose, modified cellulose, rayon, polyesters such as polyethyleneterephthalat (DACRON). The core of the absorbent layer often includes discrete particles of hydrogel materials. Such hydrogel materials are inorganic or organic compounds capable of absorbing fluids and retaining them under moderate pressures. The hydrogels can be silica gels or organic compounds such as cross-linked polymers. Examples of polymeric materials include the polyacrylamides, polyvinyl alcohol, ethylene maleic anhydride copolymers, polyvinyl ethers, hydroxypropyl cellulose, carboxymethyl cellulose etc.

### Topsheet

The topsheet which is intended for use in fabricating the wound dressing of the invention may be made in part or completely of synthetic fibers or films from such materials as polyester, modified polyolefins such as low density polyethylene and polypropylene, modified polyesters and modified poly(acrylonitriles). Examples of materials of these types include oxidized cellulosics, phosphorylated cellulosics, carboxymethylated cellulosics, grafts of cellulosics with polyacrylics, sulfonated polyolefins, partially hydrolyzed poly(acrylonitriles) and partially hydrolyzed polyesters or the like. The topsheet can also be made of natural fibers, modified celluloses such as cotton, ramie and the like. In non-woven topsheets, the fibers are bound together by a thermal bonding procedure or by polymeric binders such as polyacrylates. This sheet is substantially porous and encourages liquid materials to readily pass through into the underlying absorbent core. A suitable type of topsheet, is for example, formed from a liquid impervious polymeric material such as a polyolefin. Such topsheets can have tapered capillaries to permit flow of discharged liquid materials through the topsheet into the underlying absorbent core.

### Bottomsheet

The bottom sheet may be constructed from a thin, plastic film of polyethylene, polypropylene or other flexible material which is liquid impervious but semi-occlusive (semi-permeable) to gas.

### Odour Control Layer

The odour control layer will generally comprise an odour controlling component located within a layer of material. The component may be an anti-bacterial agent, or an odour absorbing agent. Examples of suitable odour controlling components can be found in the following documents, the relevant content of which is incorporated herein by reference: U.S. Pat. No. 4,304,675; U.S. Pat. No. 4,525,410; WO 81/01643; U.S. Pat. No. 4,826,497; WO 91/11977; and WO 91/12030.

The invention is not limited to the embodiments hereinbefore described which may be varied in construction and detail without departing from the spirit of the invention.

## Claims

1. A wound dressing for a wound of the type having a wound bed and wound exudate, the dressing comprising an absorbent core disposed on the dressing such that upon application to a wound the absorbent core will absorb wound exudate from the wound bed, the dressing incorporating a wound healing substance around the absorbent core which is disposed on the dressing such that upon application to a wound, the wound healing substance will be brought into contact with a periphery of the wound bed.

2. A wound dressing as claimed in Claim 1 in which the wound healing substance encircles the absorbent core.

3. A wound dressing as claimed in Claim 1 or 2 in which the wound healing substance is separated from the absorbent core by a liquid impermeable barrier layer.

4. A wound dressing as claimed in any preceding Claim in which the absorbent core has a wound abutting face, wherein a liquid impermeable barrier layer covers the surface of the absorbent core except for the wound abutting face.

5. A wound dressing as claimed in Claim 4 in which the liquid impermeable barrier layer is gas permeable.

6. A wound dressing as claimed in any preceding Claim further including an odour control layer.

7. A wound dressing as claimed in any preceding Claim in which the absorbent core has a central wicking portion which in use abuts the wound bed, and a main body portion located above, and in liquid communication with, the central wicking portion.

8. A wound dressing as claimed in Claim 9 in which absorbent core has a generally T-shaped profile.

9. A wound dressing as claimed in Claim 7 or 8 in which the main body portion of the absorbent core has a surface area greater then a surface area of the central wicking portion.

10. A kit for treating a wound of the type having a wound bed and wound exudate, the kit comprising:
- a wound dressing comprising an absorbent core disposed on the dressing such that upon application to a wound the absorbent core will absorb wound exudate from the wound bed;
- a wound healing substance contained within a suitable dispenser; and
- instructions for the user to apply the wound healing substance onto the wound dressing around the absorbent core such that, upon application to the wound, the wound healing substance will be brought into contact with a periphery of the wound bed.
